(19) Europäisches Patentamt
European Patent Office
Office européen des brevets

(11) **EP 3 983 800 B1**

(12) **EUROPÄISCHE PATENTSCHRIFT**

(45) Veröffentlichungstag und Bekanntmachung des Hinweises auf die Patenterteilung:
**16.04.2025 Patentblatt 2025/16**

(21) Anmeldenummer: **20731051.7**

(22) Anmeldetag: **05.06.2020**

(51) Internationale Patentklassifikation (IPC):
**G01N 33/22** (2006.01)   **G01N 27/16** (2006.01)
**F17D 1/04** (2006.01)

(52) Gemeinsame Patentklassifikation (CPC):
**G01N 33/225; F17D 1/04;** G01N 27/16

(86) Internationale Anmeldenummer:
**PCT/EP2020/065570**

(87) Internationale Veröffentlichungsnummer:
**WO 2020/249470 (17.12.2020 Gazette 2020/51)**

(54) **GASVERTEILNETZ MIT EINER MESSEINRICHTUNG ZUR BESTIMMUNG DES BRENNWERTS EINES GASSTROMS**

GAS DISTRIBUTION NETWORK HAVING A MEASURING DEVICE FOR DETERMINING THE CALORIFIC VALUE OF A GAS FLOW

RÉSEAU DE DISTRIBUTION DE GAZ COMPRENANT UN DISPOSITIF DE MESURE POUR DÉFINIR LA PUISSANCE CALORIFIQUE D'UN FLUX DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.06.2019 DE 102019115973**

(43) Veröffentlichungstag der Anmeldung:
**20.04.2022 Patentblatt 2022/16**

(73) Patentinhaber: **Westnetz GmbH
44139 Dortmund (DE)**

(72) Erfinder: **PETERS, Klaus
46483 Wesel (DE)**

(74) Vertreter: **Cohausz & Florack
Patent- & Rechtsanwälte
Partnerschaftsgesellschaft mbB
Bleichstraße 14
40211 Düsseldorf (DE)**

(56) Entgegenhaltungen:
**EP-A1- 2 045 507        EP-A1- 2 045 507
EP-A1- 2 450 704        EP-A1- 2 450 704
DE-A1- 102016 014 151   DE-A1- 102016 014 151
DE-A1- 19 921 167       DE-A1- 19 921 167
DE-T2- 69 316 643       DE-T2- 69 316 643
DE-T2- 69 316 643       FR-A1- 3 064 718
FR-A1- 3 064 718**

**Beschreibung**

Die Erfindung betrifft ein Gasverteilnetz mit einem Gasleitungssystem miteinander verbundener Gasleitungen und einer Messeinrichtung.

Messeinrichtungen zur Brennwertbestimmung von Gasströmen sind aus dem Stand der Technik grundsätzlich bekannt und werden an zentralen Einspeisestellen eines lokalen Gasverteilnetzes aus einem überregionalen Gasnetz eingesetzt, um den Brennwert des eingespeisten Gasstroms zu messen. Die Bestimmung des Brennwerts ist notwendig, um aus dem Gasvolumen (in $m^3$), das ein an das Gasverteilnetz angeschlossener Gasabnehmer aus dem Gasverteilnetz abgenommen hat und das typischerweise mit einem Volumenstrommesser am Anschluss des Gasabnehmers an das Gasverteilnetz gemessen wird, auf die damit verbundene Energiemenge (in kWh) zu schließen, auf deren Basis die Abrechnung erfolgt.

Gemäß dem heutigen Stand der Technik werden zur Brennwertbestimmung Gaschromatographen, Verbrennungs-Kalorimeter oder Absorptionsspektrometer eingesetzt. Mit einem Gaschromatographen werden die Mengenanteile der einzelnen Gasbestandteile gemessen und daraus Schlussfolgerung auf den Brennwert des Gasgemisches insgesamt gezogen. Bei einem Verbrennungs-Kalorimeter wird eine Probemenge Gas, ggf. katalytisch, verbrannt und die dabei freigesetzte Energie gemessen, aus der dann in Relation zum Gas- und Luftzustrom der Brennwert ermittelt werden kann. Mit einem Absorptionsspektrometer werden die Mengenanteile der einzelnen Gasbestandteile mit Hilfe der Lichtabsorption der Gasbestandteile ermittelt und daraus Schlussfolgerung auf den Brennwert des gesamten Gasgesmisches gezogen. Diese Messverfahren sind jedoch sehr aufwändig, so dass entsprechende Messgeräte in der Regel mehr als 30.000 EUR, ggf. sogar mehr als 100.000 EUR kosten und diese deshalb i.d.R. nur in der Großmengenmessung bzw. Transportnetzebene kostenoptimal eingesetzt werden können.

Um die Betriebs- und damit verbundenen Abrechnungskosten für ein lokales Gasversorgungsnetz gering zu halten, werden derartige Geräte aufgrund ihrer hohen Anschaffungskosten heutzutage nur an zentralen Punkten in Erdgasnetzen eingesetzt, insbesondere an den zuvor erwähnten zentralen Einspeisestellen aus einem überregionalen Gasnetz.

Weiter sind verschiedene Verfahren und Vorrichtungen zur Bestimmung eines Brennwertes oder einer Wobbezahl eines Gases bekannt aus der DE 199 21 167 A1, der DE 10 2016 014 151 A1 und der DE 693 16 643 T2.

Weiterhin offenbart die EP2045507A1 ein Gasverteilnetz mit einer dezentralen Gaseinspeiseanlage und mit einer an eine Gasleitung angeschlossenen Messeinrichtung, umfassend einen Brennwertsensor und eine Steuereinrichtung, welche die Gaseinspeisung in Abhängigkeit vom bestimmten Brennwert steuert.

Die zunehmende Dezentralisierung der Gasversorgung, insbesondere die Zunahme an lokale Gasversorgungsnetze angeschlossener dezentraler Gaseinspeiseanlagen, wie zum Beispiel Wasserstoff- oder Biogaseinspeiseanlagen, führt zu größeren Schwankungen in der Gasbeschaffenheit, insbesondere hinsichtlich der Wasserstoffkonzentration und damit hinsichtlich des Brennwerts. Der spezifische Brennwert von Wasserstoff liegt beispielsweise deutlich unter dem spezifischen Brennwert von Methan. Zudem kommt es zu höheren Schwankungen der Gasbeschaffenheit innerhalb eines lokalen Gasversorgungsnetzes, die durch die an zentralen Stellen vorgesehenen Messeinrichtungen zur Brennwertbestimmung nicht mehr ausreichend abgebildet werden können.

Vor diesem Hintergrund liegt der vorliegenden Erfindung die Aufgabe zu Grunde, die Messung der Gasbeschaffenheit auch bei dezentraler Gaseinspeisung in ein Gasverteilnetz in wirtschaftlicher Weise zu verbessern.

Diese Aufgabe wird erfindungsgemäß gelöst durch die Verwendung einer Messeinrichtung zur Bestimmung des Brennwerts eines Gasstroms in einer Gasleitung, insbesondere eines Gasverteilnetzes, gemäß Patentanspruch 1, mit einem Brennwert-Sensor, der dazu eingerichtet ist, einen Wert für den Gasbrennwert eines Gases in einer Gasleitung zu messen, und mit einer Steuereinrichtung, die dazu eingerichtet ist, aus dem gemessenen Wert für den Gasbrennwert, und optional weiteren erhaltenen Messwerten über den Gasstrom einen Wert für den Brennwert des Gasstroms zu bestimmen.

Es wurde festgestellt, dass sich durch den Einsatz von Pellistoren oder anderer, am Markt verfügbarer Gassensoren zur Detektion gasförmiger Substanzen, insbesondere Gassensoren, die thermische und/oder chemische Information des zu messenden Gases in ein elektrisch nutzbares Signal umwandeln, das in der Steuereinrichtung zur Ermittlung des Brennwertes herangezogen wird, Messeinrichtungen zur Bestimmung des Brennwerts eines Gasstroms in einer Gasleitung herstellen lassen, die deutlich kostengünstiger sind als die bisher verwendeten Gaschromatographen und Gaskalorimeter. Dies ermöglicht einen dezentralen Einsatz derartiger Messeinrichtungen zu vertretbaren Kosten, insbesondere in der Nähe dezentraler Gaseinspeiseanlagen oder in der Nähe von an das Gasverteilnetz angeschlossener Gasabnehmer, um den Brennwert des eingespeisten oder entnommenen Gases je Abnahmestelle zu bestimmen.

Bei dem Brennwert-Sensor handelt es sich entsprechend vorzugsweise um einen Pellistor oder einen Gassensor zur Detektion gasförmiger Substanzen zum Messen thermischer und/oder chemischer Informationen eines Gases. Insbesondere kann es sich bei dem Gassensor um einen Festkörper-basiertern Sensor zur Bestimmung des spezifischen Brennwerts oder der Wobbezahl handeln.

Bei der Messeinrichtung kann es sich insbesondere um ein Gerät mit einem Gehäuse handeln, in dem die Sensorik und die Steuereinrichtung angeordnet sind. Das Gehäuse ist vorzugsweise versiegelbar, beispielsweise durch eine Plombe, um Manipulationen zu verhindern. Die Messeinrichtung kann beispielsweise einen Gaseinlass und einen Gasauslass aufweisen, um den Gasfluss einer Gasleitung durch das Gerät zu leiten und kann beispielsweise über einen Lufteinlass verfügen, um einen definierten Luftstrom mit dem Erdgas zu mischen und durch den Sensor detektieren zu lassen. Des Weiteren verfügt das Gehäuse vorzugsweise über einen Gasaustritt für das am Sensor detektierte Gas-Luftgemisch, so dass das nach der Messung sicher in den Gasstrom der Kundenanlage zugeführt und dort bestimmungsgemäß genutzt werden kann, z.B. zur Wärmeerzeugung, Stromerzeugung oder Produktherstellung. Die Messeinrichtung wird vorzugsweise gemäß den geltenden Explosionsschutzanforderungen ausgeführt.

In dem Gehäuse der Messeinrichtung können insbesondere die Steuereinrichtung, der Brennwert-Sensor, ein Gassensor zur Messung der Gasfreiheit in der Umgebungsluft, eine Luftzufuhr zur definierten Luft-Gasmischung, eine Messkammer, in dem der Brennwert-Sensor, zum Beispiel der Pellistor und/oder ggf. weitere Gassensoren angeordnet sind, eine Gehäuseventilation, Schnittstellen zum Anschluss vorhandener Sensoren für eine Umgebungstemperaturmessung, Gastemperaturmessung, Volumenstrommessung, Umgebungsdruckmessung, Gasdruckmessung, Luftfeuchtigkeitsmessung und/oder Gasfeuchtigkeitsmessung oder die jeweiligen Sensoren, sofern sie nicht vorhanden sind, und/oder ein Datenspeicher, zum Beispiel für 5-Minutenwerte aller Sensoren für zwei Jahre, eine Stromversorgung, vorzugsweise über ein PV-Modul, ein Stromverteilnetz und/oder Schnittstellen zum Datenauslesen, z.B. mit WLAN,- oder Bluetooth-Standard-Schnittstellen oder GPRSbis zu 5G-Datenübertragungsstandard, untergebracht sein.

Eine Messeinrichtung mit einem Gehäuse, insbesondere in kompakter, vorzugsweise miniaturisierter Bauweise, ist insbesondere für die Installation der Messeinrichtung an einem Hausanschluss, in einer Gas-Druck-Regelanlage des Gasverteilnetzes und/oder in einer Messanlage des Gasverteilnetzes geeignet.

Die Sensoren der Messeinrichtung können alternativ auch unmittelbar in eine Gasleitung integriert sein und mit der Steuereinrichtung der Messeinrichtung verbunden sein, die in oder außerhalb der Gasleitung angeordnet sein kann. Die einzelnen Sensoren befinden sich in diesem Fall vorzugsweise in geeigneter räumlicher Nähe zueinander, insbesondere in einem Abschnitt einer Gasleitung ohne Abzweigungen zwischen den einzelnen Sensoren.

Der Pellistor weist insbesondere einen Heizdraht auf, der beim Betrieb durch Stromfluss erhitzt wird, zum Beispiel auf eine Temperatur von über 800 °C. An dem heißen Draht verbrennt das zu untersuchenden Gas und die dabei entstehende Wärme führt zu einer Veränderung der Heizdrahttemperatur, die wiederum eine Widerstandsänderung des Heizdrahtes bedingt. Durch Messen dieser Widerstandsänderung kann auf die beim Verbrennungsvorgang freigewordene Energie geschlossen und damit ein Wert für den spezifischen Brennwert oder die Wobbezahl des zu untersuchenden Gases bestimmt werden. Der Pellistor kann im Bereich des Heizdrahts einen Katalysator, zum Beispiel Platinoxid, aufweisen, um eine Verbrennung des zu untersuchenden Gases bei geringeren Heizdraht-Temperaturen, beispielsweise bei Temperaturen ab 400 °C, zu erreichen.

Neben Pellistoren eignen sich auch andere, am Markt erhältliche Gassensoren, die zur Brennwertbestimmung eines Gases geeignet sind und deutlich günstiger sind als die bisher i.d.R. an zentraler Übergabestelle vom Transportnetz in das Verteilnetz verwendeten Gaschromatographen und Gaskalorimeter.

Weiterhin wird die oben genannte Aufgabe gelöst durch ein Gasverteilnetz gemäß Patentanspruch 10, mit einem Gasleitungssystem miteinander verbundener. Gasleitungen und mit der zuvor beschriebenen Messeinrichtung oder einer Ausführungsform davon, wobei die Messeinrichtung dazu angeordnet und eingerichtet ist, den Brennwert eines Gasstroms durch eine der Gasleitungen des Gasleitungssystems zu bestimmen.

Die deutlich geringeren Kosten der zuvor beschriebenen Messeinrichtung gegenüber bisher verwendeter Gaschromatographen und Kalorimeter ermöglicht es insbesondere, die Messeinrichtungen an dezentralen Stellen im Gasverteilnetz vorzusehen, beispielsweise im Bereich einer dezentralen Einspeiseanlage, im Bereich eines (dezentralen) Gasabnehmers und/oder an Knotenpunkten des Gasleitungssytems.

Dies ermöglicht eine dezentrale Überwachung des Gases im Gasleitungssystem, so dass auch die dezentrale Gaseinspeisung von Wasserstoff- oder Biogaseinspeiseanlagen überwacht werden kann.

Als Gas wird vorliegend daher insbesondere ein Gasgemisch mit den Eigenschaften gemäß DVGW-Arbeitsblatt G260, oder ein Gemisch aus Biogas, synthetisch hergestelltem Methan oder Wasserstoff mit Gasen gemäß DVGW-Arbeitsblatt G260 verstanden. Die im Gasleitungssystem vorhandenen und mit der Messeinrichtung zu messenden Gasgemische können also erheblich von dem Gas gemäß der Spezifikationen des DVGW-Arbeitsblattes G260 abweichen. Diese Abweichung macht eine dezentrale Gasbeschaffenheitsmessung erforderlich. Die zuvor beschriebene Messeinrichtung und das Gasverteilnetz mit solchen Messeinrichtungen befähigt Erdgasverteilnetzbetreiber somit insbesondere zur Umstellung auf klimafreundlichere Gase zur Wärmeerzeugung bei Endkunden.

Im Folgenden werden verschiedene Ausführungsformen der Messeinrichtung und des Gasverteilnetzes beschrieben, wobei die einzelnen Ausführungsformen unabhängig voneinander sowohl für die Messeinrichtung als auch für das Gasverteilnetzes gelten. Die einzelnen Ausführungsformen können zudem miteinander kombiniert werden.

Bei einer Ausführungsform ist die Messeinrichtung dazu eingerichtet, einen gemessenen Wert für den Gasdruck in der Gasleitung zu erhalten, und die Steuereinrichtung ist weiter dazu eingerichtet, den Wert für den Brennwert des Gasstroms abhängig von dem gemessenen Wert für den Gasdruck zu bestimmen. Auf diese Weise kann bei der Berechnung des Brennwerts der tatsächliche aktuelle Gasdruck, insbesondere anstelle eines vorgegebenen mittleren Versorgungsdrucks, berücksichtigt werden, so dass sich der Brennwert des Gasstroms genauer ermitteln lässt.

Um einen gemessenen Wert für den Gasdruck in der Gasleitung zu erhalten, kann die Messeinrichtung insbesondere einen Drucksensor aufweisen, der dazu eingerichtet ist, einen Wert für den Gasdruck in der Gasleitung zu messen. Alternativ kann die Messeinrichtung auch eine Datenschnittstelle zum Anschluss der Messeinrichtung an einen externen Drucksensor aufweisen und die Steuereinrichtung kann dazu eingerichtet sein, den gemessenen Wert für den Gasdruck in der Gasleitung über die Datenschnittstelle zu erhalten. Auf diese Weise kann ein bereits vorhandener Drucksensor verwendet werden, so dass ein eigener Drucksensor der Messeinrichtung entbehrlich ist.

Die Steuereinrichtung der Messeinrichtung kann gemäß einer Ausführungsform dazu eingerichtet sein, den erhaltenen Wert für den Gasdruck auf das Unterschreiten eines vorgegebenen Mindestgasdrucks zu überwachen und beim Unterschreiten des Mindestgasdrucks die Ausgabe einer Warnmeldung zu bewirken. Auf diese Weise kann mit der Messeinrichtung zusätzlich eine mögliche Leckage bzw. ein Rohrleitungsbruch im bzw. eine Sabotageeingriff am Gasleitungssystem detektiert werden. Diese Funktionalität bietet sich bei den beschriebenen Messeinrichtungen insbesondere deshalb an, da dessen kostengünstige Herstellung einen Einsatz an mehreren Stellen im Gasverteilnetz ermöglicht.

Bei einer weiteren Ausführungsform ist die Messeinrichtung dazu eingerichtet, einen gemessenen Wert für die Gastemperatur in der Gasleitung zu erhalten, und die Steuereinrichtung ist weiter dazu eingerichtet, den Wert für den Brennwert des Gasstroms abhängig von dem gemessenen Wert für die Gastemperatur zu bestimmen. Auf diese Weise kann bei der Berechnung des Brennwerts die tatsächliche aktuelle Gastemperatur, insbesondere anstelle einer vorgegebenen mittleren Gastemperatur, berücksichtigt werden, so dass sich der Brennwert genauer ermitteln lässt.

Um einen gemessenen Wert für die Gastemperatur in der Gasleitung zu erhalten, kann die Messeinrichtung insbesondere einen Temperatursensor aufweisen, der dazu eingerichtet ist, einen Wert für die Gastempertaur in der Gasleitung zu messen. Alternativ kann die Messeinrichtung auch eine Datenschnittstelle zum Anschluss der Messeinrichtung an einen externen Temperatursensor aufweisen und die Steuereinrichtung kann dazu eingerichtet sein, den gemessenen Wert für die Gastemperatur in der Gasleitung über die Datenschnittstelle zu erhalten. Auf diese Weise kann ein bereits vorhandener Temperatursensor verwendet werden, so dass ein eigener Temperatursensor der Messeinrichtung entbehrlich ist.

Bei einer weiteren Ausführungsform ist die Messeinrichtung dazu eingerichtet, einen gemessenen Wert für den Gasvolumenstrom in der Gasleitung zu erhalten, und die Steuereinrichtung ist weiter dazu eingerichtet, den Wert für den Brennwert des Gasstroms abhängig von dem gemessenen Wert für den Gasvolumenstrom zu bestimmen. Auf diese Weise kann bei der Berechnung des Brennwerts der tatsächliche aktuelle Gasvolumenstrom berücksichtigt werden, so dass sich der Brennwert des aktuellen Gasvolumenstroms integriert über die mit dem Gasvolumenstrom geförderte Gasmenge ermitteln lässt.

Um einen gemessenen Wert für den Gasvolumenstrom in der Gasleitung zu erhalten, kann die Messeinrichtung insbesondere einen Volumenstrom-Sensor, zum Beispiel einen Rädchen-Zähler oder einen Balgengaszähler, aufweist, der dazu eingerichtet ist, einen Wert für den Gasvolumenstrom in der Gasleitung zu messen. Alternativ kann die Messeinrichtung auch eine Datenschnittstelle zum Anschluss der Messeinrichtung an einen Volumenstromsensor aufweisen und die Steuereinrichtung kann dazu eingerichtet sein, den gemessenen Wert für den Gasvolumenstrom in der Gasleitung über die Datenschnittstelle zu erhalten. Auf diese Weise kann ein bereits vorhandener Volumenstromsensor verwendet werden, so dass ein eigener Volumenstromsensor der Messeinrichtung entbehrlich ist.

Insbesondere weisen Hausanschlüsse grundsätzlich einen jeweiligen Volumenstromsensor auf, typischerweise einen Balgengaszähler, um zu Abrechnungszwecken die aus dem Gasverteilnetz entnommene Gasmenge zu bestimmen. Entsprechend kann die Datenschnittstelle vorzugsweise zum Anschluss an einen Volumenstromsensor eines Hausanschlusses eingerichtet sein.

Beispielsweise werden an Hausanschlüssen heutzutage sogenannte Smart-Meter installiert. Die Datenschnittstelle der Messeinrichtung kann entsprechend zum Anschluss an ein solches Smart-Meter eingerichtet sein, um über das Smart-Meter Messwerte von einem vorhandenen Volumenstromsensor zu erhalten.

Bei einer Ausführungsform weist die Messeinrichtung eine elektronische Zählwerksauswertung, insbesondere mit einer Scanner-Einheit, auf, die dazu eingerichtet ist, das Zählwerk eines analogen Gaszählers auszuwerten, um einen Wert für den Gasvolumenstrom zu erhalten.

Bei einer weiteren Ausführungsform weist die Messeinrichtung eine Datenschnittstelle zur Datenfernübertragung auf. Die Datenschnittstelle kann insbesondere zur unmittelbaren Herstellung einer Datenfernübertragungsverbindung eingerichtet sein, zum Beispiel über ein Mobilfunknetz. Alternativ kann die Datenschnittstelle auch zur mittelbaren Kommunikation über eine von einer anderen Komponente hergestellte Datenfernübertragungsverbindung eingerichtet sein, beispielsweise als Netzwerkschnittstelle zum Anschluss an ein lokales Netzwerk mit einer Internetverbindung. Die Steuereinrichtung ist vorzugsweise dazu eingerichtet, Informationen über den bestimmten Brennwert, insbesondere den bestimmten Wert für den Brennwert oder eine Information, ob der bestimmte Wert für den Brennwert einen vorgegebenen Grenzwert über- oder unterschreitet, über die Datenschnittstelle zu senden, insbesondere an einen externen Server, beispielsweise einen zentralen Server der Gasverteilnetzkontrollinstanz.

Bei einer entsprechenden Ausführungsform des Gasverteilnetzes umfasst dieses vorzugsweise einen zentralen Server, der dazu eingerichtet ist, Informationen über den von den einzelnen Messeinrichtungen des Gasverteilnetzes gemessenen Brennwert zu empfangen und das Gasverteilnetz abhängig von diesen Informationen zu steuern. Dies ermöglicht eine zentrale Netzsteuerung auf Basis der dezentral ermittelten Informationen über den jeweiligen Brennwert.

Bei einer weiteren Ausführungsform weist die Messeinrichtung einen Datenspeicher auf und die Steuereinrichtung ist dazu eingerichtet, dem jeweiligen Zeitpunkt zugeordnete Datensätze mit Informationen über den bestimmten Brennwert, insbesondere den bestimmten Wert für den Brennwert oder eine Information, ob der bestimmte Wert für den Brennwert einen vorgegebenen Grenzwert über- oder unterschreitet, auf dem Datenspeicher zu speichern. Beispielsweise kann der Datensatz einen Zeitstempel über den jeweiligen Zeitpunkt umfassen. Auf diese Weise kann die Entwicklung der ermittelten Gasbeschaffenheit zum Beispiel für eine spätere Auswertung archiviert werden. Die Steuereinrichtung kann zum Beispiel dazu eingerichtet sein, periodisch, zum Beispiel alle 5 Minuten, einen entsprechenden Datensatz zu speichern. Der Datenspeicher ist vorzugsweise so groß bemessen, dass er periodisch gespeicherte Datensätze über einen Zeitraum von mindestens 2 Jahren speichern kann.

Bei einer weiteren Ausführungsform weist die Messeinrichtung eine netzunabhängige Stromversorgung auf, zum Beispiel umfassend einen Akkumulator und optional ein Photovoltaik-Panel (PV-Panel). Auf diese Weise kann der Betrieb der Messeinrichtung auch bei Stromnetzausfällen sichergestellt werden. Weiterhin ermöglicht dies den einfachen Einsatz der Messeinrichtungen an Orten, an denen kein unmittelbarer Anschluss an das Stromnetz möglich oder sinnvoll ist.

Bei einer weiteren Ausführungsform sind die Messgenauigkeit der Sensoren der Messeinrichtung und die Berechnungsgenauigkeit der Steuereinrichtung so eingerichtet, dass sie die Messgenauigkeitsanforderungen des DVGW-Arbeitsblattes G685 und/oder des am Einsatzort gültigen Eichrechts erfüllen. Auf diese Weise können die von der Messeinrichtung bestimmten Brennwerte unmittelbar zu Abrechnungszwecken verwendet werden.

Erfindungsgemäß ist eine dezentrale Gaseinspeiseanlage an einer Gasleitung des Gasleitungssystems angeschlossen, wobei die Messeinrichtung dazu eingerichtet ist, den Brennwert des von der Gaseinspeiseanlage in das Gasleitungssystem eingespeisten Gasstroms zu bestimmen. Zu diesem Zweck ist die Messeinrichtung vorzugsweise im Bereich des Anschlusspunkts der dezentralen Gasspeiseanlage an das Gasleitungssystem angeordnet. Bei der Gaseinspeiseanlage kann es sich beispielsweise um eine dezentrale Wasserstoffeinspeiseanlage oder um eine Biogaseinspeiseanlage handeln.

Auf diese Weise lässt sich der Brennwert des dezentral in das Gasverteilnetz eingespeisten Gases bestimmen, so dass der Gasbrennwert im Gasverteilnetz dezentral überwacht werden kann. Die Steuereinrichtung ist erfindungsgemäß dazu eingerichtet, aus dem bestimmten Wert für den Brennwert eine Information über die Zusammensetzung des Gasstroms zu bestimmen, nämlich auf den Wasserstoffgehalt. Die Brennwerte von Erdgas, Biogas und Wasserstoff sind verschieden, so dass sich anhand des Brennwert der Wasserstoffgehalt und/oder der Methangehalt abschätzen, hochrechnen oder bei Anwendung einer anerkannten Kalibrierungsmethode sicher bestimmen lässt.

Die dezentrale Gaseinspeiseanlage ist erfindungsgemäß dazu eingerichtet, die Gaseinspeisung in das Gasleitungssystem abhängig von dem von der Messeinrichtung bestimmten Brennwert des Gasstroms zu steuern. Erfindungsgemäß kann die Gaseinspeisung in das Gasleitungssystem reduziert oder gestoppt werden, beispielsweise durch Abschaltung der Gaseinspeiseanlage, wenn der bestimmte Wasserstoffgehalt eine vorgegebene Höchstgrenze, zum Beispiel 30 Vol.-%, überschreitet.

Dadurch kann die Zusammensetzung des Gases im Gasverteilnetz dezentral geregelt werden. Auf diese Weise kann verhindert werden, dass sich der Wasserstoffgehalt des Gases über einen zulässigen Anteil erhöht. Bei diesem Ausführungsbeispiel kann die dezentrale Einspeisung z.B. direkt am oder in räumlicher Nähe zum Hausanschluss erfolgen. Micro-Einspeiseanlagen werden dadurch möglich. Durch die dezentrale Einspeisung des dezentral erzeugten Gases muss der Hersteller und Inverkehrbringer eine definierte Produktqualität sicherstellen und kann diese zum Beispiel durch die hier beschriebene Messeinrichtung nachweisen und durch Steuerung der Einspeisemenge des dezentral erzeugten Gases an ein festgelegtes Produktqualitätsniveau anpassen. Bei einer weiteren Ausführungsform des Gasverteilnetzes ist ein Gasabnehmer an eine Gasleitung des Gasleitungssystems angeschlossen, insbesondere über einem Hausanschluss, wobei die Messeinrichtung dazu eingerichtet ist, den Brennwert des an den Gasabnehmer geleiteten Gasstroms zu bestimmen. Auf diese Weise kann der Brennwert des an den Gasabnehmer geleiteten Gasstroms dezentral bestimmt und auf diese Weise die Genauigkeit der Abrechnung bei schwankenden Gasbrennwerten im Gasleitungssystem verbessert werden.

Bei einer weiteren Ausführungsform ist der Gasabnehmer dazu eingerichtet, die Gasabnahme aus dem Gasleitungssystem oder einen mit dem abgenommenen Gas betriebenen Verbrennungsvorgang abhängig von dem von der Messeinrichtung bestimmten Brennwert des Gasstroms zu steuern. Auf diese Weise kann der Gasabnehmer zum Beispiel genau die benötigte Brennleistung aus dem Gasverteilnetz entnehmen. Durch die Steuerung eines Verbrennungsvorgangs kann der Verbrennungsvorgang an den Gasbrennwert angepasst werden, so dass der Verbrennungsvorgang optimaler bzw. wirtschaftlicher ablaufen kann. Auf diese Weise kann beispielsweise die Verbrennungssteuerung in einer Brennwerttherme oder in einem dezentralen Block-Heizkraft-Werk optimiert werden.

Bei der Steuerung des Verbrennungsvorgangs kann zum Beispiel auch eine Abschaltung, insbesondere Notabschaltung, der Verbrennungsanlage, beispielsweise einer Heizungsanlage, erfolgen, wenn der Brennwert des Gases oder eine daraus bestimmte Beschaffenheit, nämlich der Wasserstoffgehalt, außerhalb eines vorgegebenen Betriebsbereichs liegen. Auf diese Weise können die Sicherheit der Verbrennungsanlage, zum Beispiel vor Beschädigung oder Zerstörung durch eine falsche Gasbeschaffenheit, und deren Betrieb erhöht werden.

Zur Erhöhung der Sicherheit kann die Messeinrichtung bei einer weiteren Ausführungsform einen Messsensor zur Bestimmung eines Werts für die Konzentration eines brennbaren Gases außerhalb der Gasleitung, insbesondere in der Umgebungsluft, aufweisen und die Steuereinrichtung kann dazu eingerichtet sein, die Ausgabe einer Warnmeldung zu bewirken, wenn der bestimmte Wert für die Konzentration eines brennbaren Gases außerhalb der Gasleitung einen vorgegeben Grenzwert überschreitet. Diese Ausführungsform ist besonders vorteilhaft, wenn die Messeinrichtung im Bereich eines Hausanschlusses angeordnet ist. Auf diese Weise können die Bewohner des Hauses oder eines anderen Gebäudes vor einem Gasleck gewarnt werden. Bei der Warnmeldung kann es sich beispielsweise um einen Signalton oder eine Warn-SMS handeln, die zum Beispiel über einen SMS-Dienst versandt wird, wenn die Messeinrichtung über eine Datenschnittstelle eine entsprechendes Warnsignal ausgibt.

Weitere Merkmale und Vorteile der Messeinrichtung und des Gasverteilnetzes ergeben sich aus der nachfolgenden Beschreibung von Ausführungsbeispielen, wobei auf die beigefügte Zeichnung Bezug genommen wird.

In der Zeichnung zeigen

Fig. 1 ein lokales Gasverteilnetz aus dem Stand der Technik,

Fig. 2 ein erstes Ausführungsbeispiel der Messeinrichtung,

Fig. 3 ein zweites Ausführungsbeispiel der Messeinrichtung,

Fig. 4 ein erstes Ausführungsbeispiel des Gasverteilnetzes und

Fig. 5 ein zweites Ausführungsbeispiel des Gasverteilnetzes.

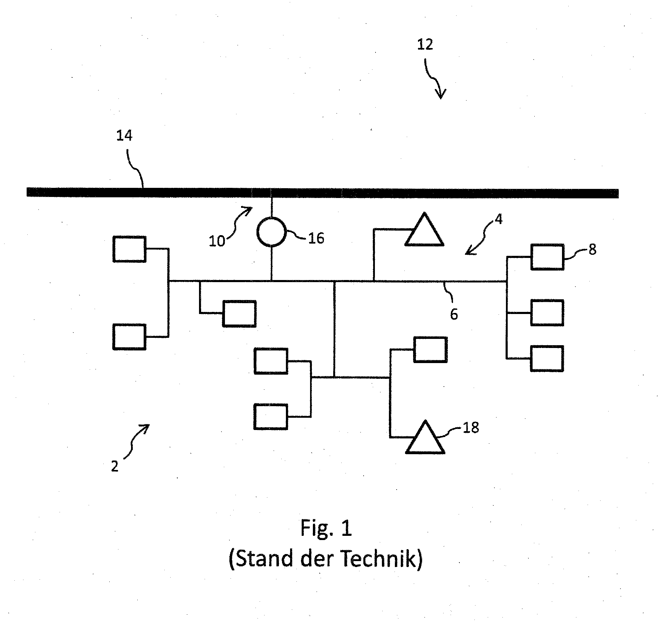

Fig. 1 zeigt ein lokales Gasverteilnetz aus dem Stand der Technik. Das Gasverteilnetz 2 umfasst ein Gasleitungssystem 4 aus miteinander verbundenen Gasleitungen 6, an die verschiedene Gasabnehmer 8 (Rechtecke) angeschlossen sind. Das Gasverteilnetz 2 ist an einer zentralen Einspeisestelle 10 mit einem überregionalen Gasnetz 12 verbunden, von dem in Fig. 1 eine Ferngasleitung 14 dargestellt ist.

Die Beschaffenheit des aus der Ferngasleitung 14 in das Gasverteilnetz 2 eingespeisten Gases wird durch eine Messeinrichtung 16 (Kreis) bestimmt. Zur Bestimmung des Brennwerts des eingespeisten Gases umfasst die zentrale Messeinrichtung 16 einen Gaschromatographen oder ein Kalorimeter. Da diese Geräte sehr teuer sind, ist in dem Gasverteilnetz 2 nur eine einzige Messeinrichtung 16 im Bereich der zentralen Einspeisestelle 10 vorgesehen.

In Gasverteilnetzen, die allein zentral aus dem überregionalen Gasnetz 12 gespeist werden, reicht eine einzige Messeinrichtung 16 im Bereich der zentralen Einspeisestelle 10 typischerweise aus, um die Gasbeschaffenheit, insbesondere den Brennwert im lokalen Gasverteilnetz 2 ausreichend genau zu bestimmen.

In jüngerer Zeit werden jedoch vermehrt dezentrale Einspeiseanlagen 18 (Dreiecke) an lokale Gasverteilnetze angeschlossen, wie zum Beispiel Wasserstoff- oder Biogaseinspeiseanlagen, die die Beschaffenheit des Gases im Gasverteilnetz ändern. Durch die dezentrale Einspeisung kann es sogar zur selben Zeit zu unterschiedlichen Gasbeschaffenheiten in verschiedenen Bereichen des Gasverteilnetzes kommen.

Eine einzelne Messeinrichtung 16 an der zentralen Einspeisestelle 10 wird in diesen Fällen regelmäßig nicht mehr ausreichen, um die Gasbeschaffenheit im Gasverteilnetz ausreichend genau zu bestimmen, so dass es zum Beispiel zu fehlerhaften Abrechnungen gegenüber den Gasabnehmern kommen kann.

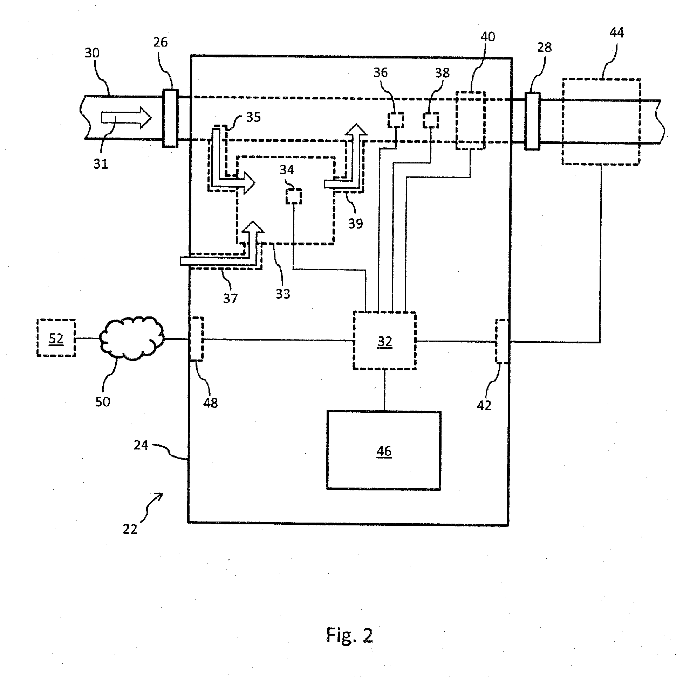

Fig. 2 zeigt ein erstes Ausführungsbeispiel der Messeinrichtung in schematischer Darstellung. Die Messeinrichtung 22 weist ein Gehäuse 24 mit einem Gaseinlass 26 und einem Gasauslass 28 auf. Die Messeinrichtung 22 ist mit dem Gaseinlass 26 und dem Gasauslass 28 so an eine Gasleitung 30 anschließbar bzw.

angeschlossen, dass ein in der Gasleitung 30 fließender Gasstrom 31 durch die Messeinrichtung 22 geführt wird.

In dem Gehäuse 24 sind eine Steuereinrichtung 32 und mehrere Sensoren untergebracht, nämlich ein Brennwert-Sensor 34, ein Drucksensor 36, ein Temperatursensor 38 und ein Volumenstromsensor 40, die mit der Steuereinrichtung 32 verbunden sind. Die Sensoren 34, 36, 38 und 40 sind so angeordnet, um Werte für den Brennwert, den Gasdruck, die Gastemperatur bzw. den Gasvolumenstrom des durch das Gehäuse 24 geleiteten Gasstroms 31 zu messen. Hierbei sind die Sensoren 36, 38 und 40 direkt in dem im Betrieb durch die Messeinrichtung 22 geführten Gasstrom 31 angeordnet. Der Brennwert-Sensor 34 ist demgegenüber vorzugsweise nicht direkt im Gasstrom 31 angeordnet, sondern in einer im Gehäuse 24 untergebrachten Messkammer 33. Im Betrieb gelangt Gas des Gasstroms 31 über eine vorzugsweise mit einem ansteuerbaren Ventil (nicht dargestellt) geregelte Zuleitung 35 in die Messkammer 33, so dass der Brennwert-Sensor 34 unter kontrollierten Umgebungsbedingungen einen Wert für die Wobbezahl bzw. für den spezifischen Brennwert des Gases bestimmen kann. Wird als Brennwert-Sensor 34 ein Pellistor eingesetzt, so erfordert dieser typischerweise ein definiertes Gas-Luftgemisch für eine genaue Brennwert-Bestimmung. Dieses definierte Gas-Luftgemisch kann in der Messkammer 33 gezielt über die Steuerung der Zuleitung 35 sowie einer weiterhin vorgesehenen und ebenfalls steuerbaren Luft-Zuleitung 37 eingestellt werden. Nach der Messung kann das Gas-Luftgemisch über eine Ableitung 39 wieder dem Gasstrom 31 zugeführt werden.

Es ist auch denkbar, auf bereits vorhandene Sensoren zuzugreifen. Zu diesem Zweck weist die Messeinrichtung 22 eine Datenschnittstelle 42 auf, die mit einem außerhalb der Messeinrichtung 22 angeordneten Sensor 44 verbindbar ist, so dass ein mit dem Sensor 44 gemessener Wert über die Datenschnittstelle 42 von der Steuereinrichtung 32 erhalten werden kann.

Wird die Messeinrichtung 22 beispielsweise bei einem Hausanschluss installiert, so kann es sich bei dem Sensor 44 beispielsweise um den Volumenstromsensor des Hausanschlusses handeln. Auf diese Weise ist der Volumenstromsensor 40 der Messeinrichtung entbehrlich, so dass die Messeinrichtung 22 kompakter und kostengünstiger hergestellt werden kann.

Bei dem Brennwert-Sensor 34 kann es sich insbesondere um einen Pellistor oder um einen anderen Gassensor zur Detektion gasförmiger Substanzen, insbesondere um einen Gassensor, der thermische und/oder chemische Information des zu messenden Gases in ein elektrisch nutzbares Signal umwandelt, handeln. Derartige Sensoren sind sehr kompakt und preisgünstig, so dass sich die Herstellungskosten für die Messeinrichtung 22 gegenüber der aus dem Stand der Technik bekannten Messeinrichtung 16 aus Fig. 1 drastisch senken lassen.

Die Steuereinrichtung 32 ist dazu eingerichtet, aus dem von dem Brennwert-Sensor 34 gemessenen Wert für den Gasbrennwert, wie zum Beispiel die Wobbezahl, aus dem von dem Drucksensor 36 gemessenen Wert für den Gasdruck p, aus dem von dem Temperatursensor 38 gemessenen Wert für die Gastemperatur T und aus dem von dem Volumenstromsensor 40 oder alternativ 44 gemessenen Wert für den Gasvolumenstrom dV/dt einen Wert für den Brennwert des Gasstroms 31 zu bestimmen.

Wenn der Brennwert-Sensor 34 zum Beispiel einen Wert für die Wobbezahl $W_S$ misst, so kann der Brennwert $H_S$ des Gases wie folgt berechnet werden:

$$H_S = W_S \cdot \sqrt{(\rho(T, p)/\rho_0(T, p))},$$

wobei $\rho$ die Gasdichte des Gases in der Gasleitung und $\rho_0$ die Gasdichte trockener Luft unter den gleichen Druck- und Temperaturbedingungen wie in der Gasleitung ist. Die Gasdichte $\rho(T, p)$ ist eine Funktion der Gastemperatur T und des Gasdrucks p und wird von der Steuereinrichtung abhängig von der gemessenen Gastemperatur und dem gemessenen Gasdruck bestimmt. Der Zusammenhang zwischen Gastemperatur, Gasdruck und Gasdichte $\rho(T, p)$ kann zum Beispiel als Formel oder als Tabelle in einem Speicher der Steuereinrichtung 32 gespeichert sein. Ebenso ist die Gasdichte $\rho_0(T, p)$ eine Funktion der Gastemperatur T und des Gasdrucks p und kann ebenfalls als Formel oder als Tabelle in einem Speicher der Steuereinrichtung 32 gespeichert sein.

Aus dem Brennwert $H_S$ des Gases und dem Volumenstrom dV/dt lässt sich dann die Brennleistung des Gasstroms 31 berechnen.

Die Messeinrichtung 22 kann weiterhin eine Nutzerschnittstelle 46, zum Beispiel mit einem Bildschirm, aufweisen, über den der berechnete Brennwert des Gases oder die Brennleistung des Gasstroms 31 angezeigt werden kann.

Weiterhin kann die Messeinrichtung 22 eine Datenschnittstelle 48 zum Anschluss an ein Netzwerk 50 aufweisen, um zum Beispiel den Brennwert oder die Brennleistung des Gasstroms 31 an einen zentralen Server 52, zum Beispiel zu Abrechnungszwecken, übermitteln zu können.

In dem Gehäuse 24 können also insbesondere die Steuereinrichtung 32, die Sensoren 34, 35, 38, 40, die Luft-Zuleitung 37 zur definierten Luft-Gasmischung, die Messkammer 33 mit dem Brennwert-Sensor 34, insbesondere Pellistor, oder sonstigen Gassensoren, ein Gassensor zur Messung der Gasfreiheit in der Umgebungsluft, eine Gehäuseventilation, Schnittstellen zum Anschluss vorhandener Sensoren für die Umgebungstemperaturmessung, Gastemperaturmessung, Volumenstrommessung, Umgebungsdruckmessung, Gasdruckmessung, Luftfeuchtigkeitsmessung Gasfeuchtigkeitsmessung oder die jeweiligen Sensoren, sofern sie nicht vorhanden sind und ein Datenspeicher für 5-Minutenwerte aller Sensoren für zwei Jahre, eine Stromversorung, z.B. über PV-Modul, und ein Stromverteilnetz, Schnittstellen zum Datenauslesen z.B. mit WLAN,- oder Bluetooth-Standard-Schnittstellen oder GPRS- bis zu 5G-Datenübertragungsstandard untergebracht sein.

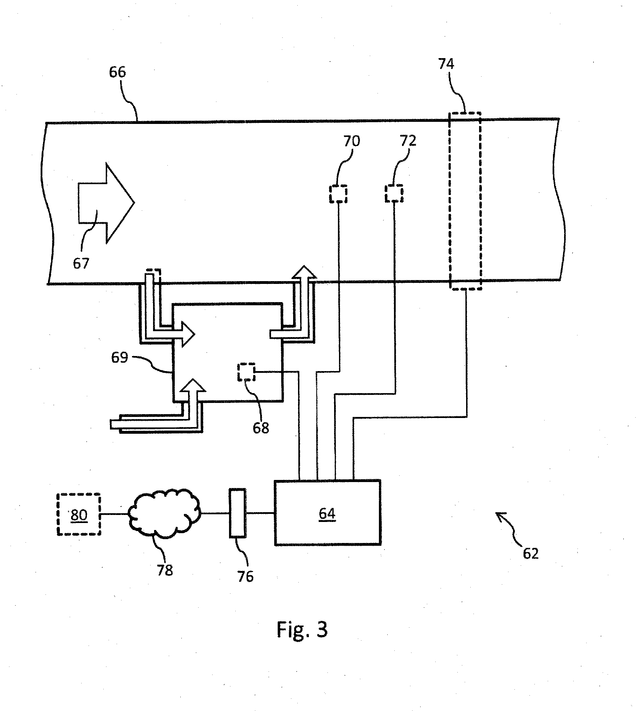

Fig. 3 zeigt ein zweites Ausführungsbeispiel der Messeinrichtung in schematischer Darstellung. Die Messeinrichtung 62 ist an einer Gasleitung 66 eines Gasverteilnetzes vorgesehen und umfasst eine Steuereinrichtung 64, einen Brennwert-Sensor 68, der in einer an die Gasleitung 66 angeschlossenen Messkammer 69 angeordnet ist, sowie weitere Sensoren, nämlich einen Drucksensor 70, einen Temperatursensor 72 und einen Volumenstromsensor 74. Bei dem Brennwert-Sensor 68 kann es sich um einen Pellistor oder um einen anderen Gassensor zur Detektion gasförmiger Substanzen, insbesondere um einen Gassensor, der thermische und/oder chemische Information des zu messenden Gases in ein elektrisch nutzbares Signal umwandelt, handeln.

Im Betrieb erhält die Steuereinrichtung 64 von dem Brennwert-Sensor 68 einen Wert für die Wobbezahl $W_S$ des in der Gasleitung 66 geleiteten Gasstroms 67, von dem Drucksensor 70 einen Wert für den Gasdruck p in der Gasleitung 66, von dem Temperatursensor 72 einen Wert für die Gastemperatur T des Gasstroms 67 in der Gasleitung 66 und von dem Volumenstromsensor 74 einen Wert für den Volumenstrom dV/dt des durch die Gasleitung 66 fließenden Gasstroms 67 und berechnet daraus einen Wert für den Brennwert $H_S$ des Gasstroms 67.

Die Steuereinrichtung sendet eine Information über den berechneten Brennwert $H_S$ , beispielsweise dessen Wert oder eine Information, ob ein vorgegebener unterer Grenzwert unterschritten oder ein oberer Grenzwert überschritten wird, über eine mit einem Netzwerk 78 verbundene Datenschnittstelle 76 der Messeinrichtung 62 an einen Server 80, zum Beispiel einen zentralen Server 80 des lokalen Gasverteilnetzes, der die Gasbeschaffenheit im lokalen Gasverteilnetz überwacht.

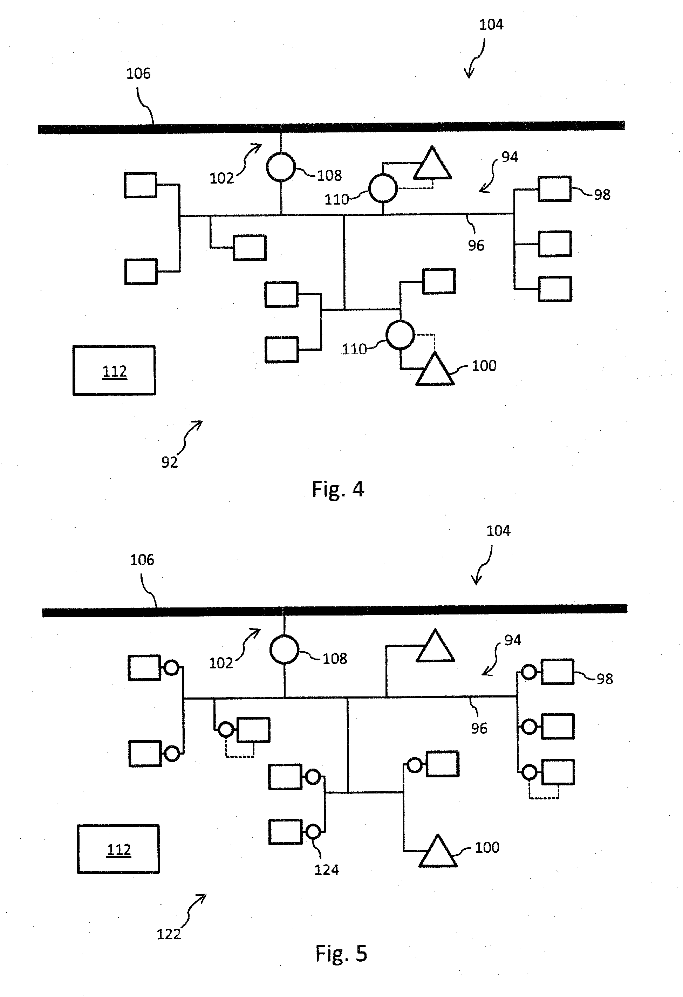

Fig. 4 zeigt ein erstes Ausführungsbeispiel des Gasverteilnetzes in schematischer Darstellung. Das Gasverteilnetz 92 umfasst ein Gasleitungssystem 94 aus miteinander verbundenen Gasleitungen 96, an die verschiedene Gasabnehmer 98 (Rechtecke) und dezentrale Einspeiseanlagen 100 (Dreiecke) angeschlossen sind. Das Gasverteilnetz 92 ist an einer zentralen Einspeisestelle 102 mit einem überregionalen Gasnetz 104 verbunden, von dem in Fig. 4 eine Ferngasleitung 106 dargestellt ist.

An der zentralen Einspeisestelle 102 ist eine zentrale Messeinrichtung 108 vorgesehen, die die Beschaffenheit, insbesondere den Brennwert, des aus der Ferngasleitung 106 in das Gasverteilnetz 92 eingespeisten Gases misst.

Zusätzlich sind im Bereich der dezentralen Einspeiseanlagen 100 dezentrale Messeinrichtungen 110 vorgesehen, die die Beschaffenheit, insbesondere den Brennwert, des von den dezentralen Einspeiseanlagen 100 in das Gasverteilnetz 92 eingespeisten Gases misst.

Die dezentralen Messeinrichtungen 110 weisen einen Aufbau auf wie die Messeinrichtungen 22 oder 62 aus Fig. 2 und 3 und sind damit deutlich kostengünstiger und kompakter als die im Stand der Technik verwendeten Messeinrichtungen 16 aus Fig. 1. Dadurch können in dem Gasverteilnetz 92 mehrere solcher Messeinrichtungen 22 eingesetzt werden, ohne dass das Gasverteilnetz 92 unwirtschaftlich wird.

Die zentrale Messeinrichtung 108 kann wie die aus dem Stand der Technik bekannte zentrale Messeinrichtung 16 mit einem Gaschromatographen oder einem Kalorimeter aufgebaut sein oder - zur weiteren Kostenersparnis - ebenfalls mit einer Messeinrichtung entsprechend Fig. 2 oder 3.

Indem in dem Gasverteilnetz 92 mehrere Messeinrichtungen 108, 110 vorgesehen sind, insbesondere an den dezentralen Einspeiseanlagen 100, kann die Beschaffenheit des Gases im Gasverteilnetz 92 besser überwacht werden. Insbesondere können durch die Beschaffenheitsmessungen an den von den dezentralen Einspeiseanlagen 100 eingespeisten Gasströmen bereichsweise Abweichungen der Gasbeschaffenheit festgestellt bzw. berechnet werden.

Zur Überwachung der Gasbeschaffenheit in den einzelnen Bereichen des Gasverteilnetzes 92 sind die Messeinrichtungen 108, 110 vorzugsweise dazu eingerichtet, Informationen über die gemessenen Brennwerte an einen zentralen Server 112 zu übermitteln. Die bereichsweise Berechnung der Gasbeschaffenheit kann dann anhand der übermittelten Informationen auf dem Server 112 erfolgen. Weiterhin kann der Server 112 Informationen über die Gasbeschaffenheit zu Abrechnungszwecken zur Verfügung stellen oder bei Abweichungen der Gasbeschaffenheit im Gasverteilnetz 92 die Ausgabe einer Warnmeldung bewirken.

Die Überwachung der Beschaffenheit der von den dezentralen Einspeiseanlagen 100 eingespeisten Gasströme ermöglicht zudem eine Steuerung der Einspeisung, wenn die Gasbeschaffenheit außerhalb eines zulässigen Bereichs liegt, nämlich zuviel Wasserstoff enthält. Zu diesem Zweck kann eine dezentrale Einspeiseanlage 100 zum Beispiel eine Information über die Gasbeschaffenheit von der jeweiligen Messeinrichtung 110 erhalten (in Fig. 4 illustriert durch die gestrichelte Linie zwischen Messeinrichtung 110 und dezentraler Einspeiseanlage 100) und die Einspeisung abhängig davon steuern, ggf. sogar stoppen, wenn der Wasserstoffgehalt oberhalb eines vorgegebenen Grenzwerts liegt. Die Steuerung der dezentralen Einspeiseanlagen 100 kann auch über den zentralen Server 112 erfolgen.

Fig. 5 zeigt ein zweites Ausführungsbeispiel des Gasverteilnetzes in schematischer Darstellung. Das Gasverteilnetz 122 weist einen ähnlichen Aufbau auf wie das Gasverteilnetz 92. Einander entsprechende Komponenten sind mit denselben Bezugszeichen versehen.

Bei dem Gasverteilnetz 122 sind anstelle der dezentralen Messeinrichtungen 110 an den dezentralen Einspeiseanlagen 100 jeweilige dezentrale Messeinrichtungen 124 (kleine Kreise) an den einzelnen Gasabnehmern 98 vorgesehen. Es ist aber auch denkbar, dass sowohl dezentrale Messeinrichtungen 110 an den dezentralen Einspeiseanlagen 100 als auch dezentrale Messeinrichtungen 124 an den Gasabnehmern 98 vorgesehen sind.

Die dezentralen Messeinrichtungen 124 weisen einen Aufbau auf wie die Messeinrichtungen 22 oder 62 aus Fig. 2 und 3 und sind damit deutlich kostengünstiger und kompakter als die im Stand der Technik verwendeten Messeinrichtungen 16 aus Fig. 1. Dadurch können in dem Gasverteilnetz 92 mehrere solcher Messeinrichtungen 22, ggf. sogar im Wesentlichen bei jedem Gasabnehmer, eingesetzt werden, ohne dass das Gasverteilnetz 92 unwirtschaftlich wird.

Bei einigen der Gasabnehmer 98 kann es sich zum Beispiel um Haushalte handeln. In diesem Fall können die zugehörigen dezentralen Messeinrichtungen 124 im Bereich der Hausanschlüsse dieser Haushalte angeordnet sein. Da Hausanschlüsse zu Abrechnungszwecken über Volumenstromsensoren verfügen, kann die dezentrale Messeinrichtung 124 zum Beispiel wie in Fig. 2 gezeigt über eine Datenschnittstelle 42 mit dem vorhandenen Volumenstromsensor verbunden werden, so dass ein eigener Volumenstromsensor der dezentralen Messeinrichtung 124 entbehrlich ist und die Geräte damit noch kostengünstiger hergestellt werden können.

Durch die dezentralen Messeinrichtungen 124 im Bereich der einzelnen Gasabnehmer 98 kann die Gasbeschaffenheit, insbesondere der Brennwert, des von einem Gasabnehmer 98 dem Gasverteilnetz 122 entnommenen Gasstroms unmittelbar gemessen werden, so dass eine genaue Abrechnung nach entnommenem Brennwert ermöglicht wird. Zu diesem Zweck können die einzelnen dezentralen Messeinrichtungen 124 Informationen über die Gasbeschaffenheit zum Beispiel an den zentralen Server 112 übermitteln.

Die Überwachung der Beschaffenheit der von den Gasabnehmern 98 entnommenen Gasströmen ermöglicht es zudem, einen Verbrennungsprozess beim Gasabnehmer an die Gasbeschaffenheit anzupassen. Zu diesem Zweck kann ein Gasabnehmer 98 zum Beispiel eine Information über die Gasbeschaffenheit von der jeweiligen Messeinrichtung 124 erhalten (in Fig. 4 illustriert durch gestrichelte Linien zwischen einigen Messeinrichtung 124 und jeweiligen Gasabnehmern 98) und den Verbrennungsprozess abhängig davon steuern, ggf. sogar stoppen, zum Beispiel wenn der Wasserstoffgehalt oberhalb eines vorgegebenen Grenzwerts liegt. Die Steuerung der Verbrennungsprozesse bei den Gasabnehmern 98 kann auch über den zentralen Server 112 erfolgen.

**Patentansprüche**

1. Verwendung einer Messeinrichtung (22, 62, 108, 110, 124) zur Bestimmung des Brennwerts eines Gasstroms (31, 67) in einer Gasleitung (30, 66, 96) eines Gasverteilnetzes (92, 122),

   - wobei die Messeinrichtung einen Brennwert-Sensor (34, 68) aufweist, der dazu eingerichtet ist, einen Wert für den Gasbrennwert eines Gases in einer Gasleitung (30, 66, 96) zu messen, und
   - wobei die Messeinrichtung eine Steuereinrichtung (32, 64) aufweist, die dazu eingerichtet ist, aus dem gemessenen Wert für den Gasbrennwert und optional weiteren erhaltenen Messwerten über den Gasstrom (31, 67) einen Wert für den Brennwert des Gasstroms (31, 67) zu bestimmen,
   - wobei das Gasverteilnetz (92, 122) ein Gasleitungssystem (94) miteinander verbundener Gasleitungen (30, 66, 96) aufweist, und
   - wobei die Messeinrichtung (22, 62, 108, 110, 124) dazu angeordnet und eingerichtet ist, den Brennwert eines Gasstroms (31, 67) durch eine der Gasleitungen (30, 66, 96) des Gasleitungssystems (94) zu bestimmen,
   **dadurch gekennzeichnet,**
   - **dass** eine dezentrale Gaseinspeiseanlage (100) an einer Gasleitung (30, 66, 96) des Gasleitungssystems (94) angeschlossen ist, wobei die Messeinrichtung (22, 62, 110, 124) dazu eingerichtet ist, den Brennwert des von der dezentralen Gaseinspeiseanlage (100) in das Gasleitungssystem (94) eingespeisten Gasstroms (31, 67) zu bestimmen,
   - **dass** die Steuereinrichtung dazu eingerichtet ist, aus dem bestimmten Wert für den Brennwert eine Information über die Zusammensetzung des Gasstroms zu bestimmen, nämlich über den Wasserstoffgehalt, und
   - **dass** die dezentrale Gaseinspeiseanlage (100) dazu eingerichtet ist, die Gaseinspeisung in das Gasleitungssystem (94) abhängig von dem von der Messeinrichtung (22, 62, 110, 124) bestimmten Brennwert des Gasstroms zu steuern, nämlich die Gaseinspeisung in das Gasleitungssystem zu reduzieren oder zu stoppen, wenn der aus dem bestimmten Wert für den Brennwert bestimmte Wasserstoffgehalt eine vorgegebene Höchstgrenze überschreitet.

2. Verwendung nach Anspruch 1,
   **dadurch gekennzeichnet,**

   - **dass** als Brennwert-Sensor (34, 68) ein Pellistor oder ein anderer Gassensor zur Detektion gasförmiger Substanzen, insbesondere einen Gassensor, der thermische und/oder chemische Information des zu messenden Gases in ein elektrisch nutzbares Signal umwandelt, verwendet wird.

3. Verwendung nach Anspruch 1 oder 2,
   **dadurch gekennzeichnet,**

   - **dass** die Messeinrichtung (22, 62, 108, 110, 124) dazu eingerichtet ist, einen gemessenen Wert für den Gasdruck in der Gasleitung (30, 66, 96) zu erhalten, und
   - **dass** die Steuereinrichtung (32, 64) weiter dazu eingerichtet ist, den Wert für den Brennwert des Gasstroms (31, 67) abhängig von dem gemessenen Wert für den Gasdruck zu bestimmen.

4. Verwendung nach Anspruch 3,
   **dadurch gekennzeichnet,**

   - **dass** die Messeinrichtung (22, 62, 108, 110, 124) einen Drucksensor aufweist, der dazu eingerichtet ist, einen Wert für den Gasdruck in der Gasleitung (30, 66, 96) zu messen.

5. Verwendung nach einem der Ansprüche 1 bis 4,
   **dadurch gekennzeichnet,**

   - **dass** die Messeinrichtung (22, 62, 108, 110, 124) dazu eingerichtet ist, einen gemessenen Wert für die Gastemperatur in der Gasleitung (30, 66, 96) zu erhalten, und
   - **dass** die Steuereinrichtung (32, 64) weiter dazu eingerichtet ist, den Wert für den Brennwert des Gasstroms (31, 67) abhängig von dem gemessenen Wert für die Gastemperatur zu bestimmen.

6. Verwendung nach Anspruch 5,
   **dadurch gekennzeichnet,**

   - **dass** die Messeinrichtung (22, 62, 108, 110, 124) einen Temperatursensor (38, 72) aufweist, der dazu eingerichtet ist, einen Wert für die Gastemperatur in der Gasleitung (30, 66, 96) zu messen.

7. Verwendung nach einem der Ansprüche 1 bis 6,
   **dadurch gekennzeichnet,**

   - **dass** die Messeinrichtung (22, 62, 108, 110, 124) dazu eingerichtet ist, einen gemessenen Wert für den Gasvolumenstrom in der Gasleitung (30, 66, 96) zu erhalten, und
   - **dass** die Steuereinrichtung (32, 64) weiter dazu eingerichtet ist, den Wert für den Brennwert des Gasstroms (31, 67) abhängig von dem gemessenen Wert für den Gasvolumenstrom zu bestimmen.

8. Verwendung nach Anspruch 7,
   **dadurch gekennzeichnet,**

   - **dass** die Messeinrichtung (22, 62, 108, 110, 124) einen Volumenstromsensor (40, 74) aufweist, der dazu eingerichtet ist, einen Wert für den Gasvolumenstrom in der Gasleitung (30, 66, 96) zu messen.

9. Verwendung nach Anspruch 8,
   **dadurch gekennzeichnet,**

   - **dass** die Messeinrichtung (22, 62, 108, 110, 124) eine Datenschnittstelle (42) zum Anschluss der Messeinrichtung (22, 62, 108, 110, 124) an einen Volumenstromsensor (44) eines Hausanschlusses aufweist und
   - **dass** die Steuereinrichtung (32, 64) dazu eingerichtet ist, den gemessenen Wert für den Gasvolumenstrom in der Gasleitung (30, 66, 96) über die Datenschnittstelle (42) zu erhalten.

10. Gasverteilnetz (92, 122),

    - mit einem Gasleitungssystem (94) miteinander verbundener Gasleitungen (30, 66, 96) und
    - mit einer Messeinrichtung (22, 62, 108, 110, 124) zur Bestimmung des Brennwerts eines Gasstroms (31, 67) in einer Gasleitung (30, 66, 96),
    - wobei die Messeinrichtung einen Brennwert-Sensor (34, 68) aufweist, der dazu eingerichtet ist, einen Wert für den Gasbrennwert eines Gases in einer Gasleitung (30, 66, 96) zu messen,
    - wobei die Messeinrichtung eine Steuereinrichtung (32, 64) aufweist, die dazu eingerichtet ist, aus dem gemessenen Wert für den Gasbrennwert und optional weiteren erhaltenen Messwerten über den Gasstrom (31, 67) einen Wert für den Brennwert des Gasstroms (31, 67) zu bestimmen, und
    - wobei die Messeinrichtung (22, 62, 108, 110, 124) dazu angeordnet und eingerichtet ist, den Brennwert eines Gasstroms (31, 67) durch eine der Gasleitungen (30, 66, 96) des Gasleitungssystems (94) zu bestimmen,
    **dadurch gekennzeichnet,**
    - **dass** eine dezentrale Gaseinspeiseanlage (100) an einer Gasleitung (30, 66, 96) des Gasleitungssystems (94) angeschlossen ist, wobei die Messeinrichtung (22, 62, 110, 124) dazu eingerichtet ist, den Brennwert des von der dezentralen Gaseinspeiseanlage (100) in das Gasleitungssystem (94) eingespeisten Gasstroms (31, 67) zu bestimmen,
    - **dass** die Steuereinrichtung dazu eingerichtet ist, aus dem bestimmten Wert für den Brennwert eine Information über die Zusammensetzung des Gasstroms zu bestimmen, nämlich über den Wasserstoffgehalt, und
- **dass** die dezentrale Gaseinspeiseanlage (100) dazu eingerichtet ist, die Gaseinspeisung in das Gasleitungssystem (94) abhängig von dem von der Messeinrichtung (22, 62, 110, 124) bestimmten Brennwert des Gasstroms zu steuern, nämlich die Gaseinspeisung in das Gasleitungssystem zu reduzieren oder zu stoppen, wenn der aus dem bestimmten Wert für den Brennwert bestimmte Wasserstoffgehalt eine vorgegebene Höchstgrenze überschreitet.

11. Gasverteilnetz nach Anspruch 10,
**dadurch gekennzeichnet, dass** ein Gasabnehmer (98) an eine Gasleitung (30, 66, 96) des Gasleitungssystems (94) angeschlossen ist, insbesondere über einen Hausanschluss, wobei die Messeinrichtung (22, 62, 110, 124) dazu eingerichtet ist, den Brennwert des an den Gasabnehmer (98) geleiteten Gasstroms (31, 67) zu bestimmen.

12. Gasverteilnetz nach Anspruch 11,
**dadurch gekennzeichnet, dass** der Gasabnehmer (98) dazu eingerichtet ist, die Gasabnahme aus dem Gasleitungssystem (94) oder einen mit dem abgenommenen Gas betriebenen Verbrennungsvorgang abhängig von dem von der Messeinrichtung (22, 62, 110, 124) bestimmten Brennwert des Gasstroms zu steuern.

## Claims

1. Use of a measuring device (22, 62, 108, 110, 124) for determining the calorific value of a gas flow (31, 67) in a gas line (30, 66, 96) of a gas distribution network (92, 122),

    - wherein the measuring device comprises a calorific value sensor (34, 68) which is configured to measure a value for the gas calorific value of a gas in a gas line (30, 66, 96), and
    - wherein the measuring device has a control device (32, 64) which is configured to determine a value for the calorific value of the gas flow (31, 67) from the measured value for the gas calorific value and optionally further obtained measured values relating to the gas flow (31, 67),
    - wherein the gas distribution network (92, 122) comprises a gas line system (94) of interconnected gas lines (30, 66, 96), and
    - wherein the measuring device (22, 62, 108, 110, 124) is arranged and configured to determine the calorific value of a gas flow (31, 67) through one of the gas lines (30, 66, 96) of the gas line system (94),
    **characterised**

    - **in that** a decentralised gas feed-in system (100) is connected to a gas line (30, 66, 96) of the gas line system (94), wherein the measuring device (22, 62, 110, 124) is configured to determine the calorific value of the gas flow (31, 67) fed into the gas line system (94) by the decentralised gas feed-in system (100),
    - **in that** the control device is configured to determine information about the composition of the gas flow, namely about the hydrogen content, from the determined value for the calorific value, and
    - **in that** the decentralised gas feed-in system (100) is configured to control the gas feed into the gas line system (94) as a function of the calorific value of the gas flow determined by the measuring device (22, 62, 110, 124), namely to reduce or stop the gas feed into the gas line system if the hydrogen content determined from the determined value for the calorific value exceeds a predetermined maximum limit.

2. Use according to claim 1,
**characterised**

    - **in that** a pellistor or another gas sensor for detecting gaseous substances, in particular a gas sensor which converts thermal and/or chemical information of the gas to be measured into an electrically usable signal, is used as the calorific value sensor (34, 68).

3. Use according to claim 1 or 2,
**characterised**

    - **in that** the measuring device (22, 62, 108, 110, 124) is configured to obtain a measured value for the gas pressure in the gas line (30, 66, 96), and
    - **in that** the control device (32, 64) is further configured to determine the value for the calorific value of the gas flow (31, 67) as a function of the measured value for the gas pressure.

4. Use according to claim 3,
**characterised**

    - **in that** the measuring device (22, 62, 108, 110, 124) has a pressure sensor which is configured to measure a value for the gas pressure in the gas line (30, 66, 96).

5. Use according to one of claims 1 to 4,
**characterised**

    - **in that** the measuring device (22, 62, 108, 110, 124) is configured to obtain a measured value for the gas temperature in the gas line (30, 66, 96), and

- **in that** the control device (32, 64) is further configured to determine the value for the calorific value of the gas flow (31, 67) as a function of the measured value for the gas temperature.

6. Use according to claim 5, **characterised**

  - **in that** the measuring device (22, 62, 108, 110, 124) has a temperature sensor (38, 72) which is configured to measure a value for the gas temperature in the gas line (30, 66, 96).

7. Use according to one of claims 1 to 6, **characterised**

  - **in that** the measuring device (22, 62, 108, 110, 124) is configured to obtain a measured value for the gas volume flow in the gas line (30, 66, 96), and
  - **in that** the control device (32, 64) is further configured to determine the value for the calorific value of the gas flow (31, 67) as a function of the measured value for the gas volume flow.

8. Use according to claim 7, **characterised**

  - **in that** the measuring device (22, 62, 108, 110, 124) has a volume flow sensor (40, 74) which is configured to measure a value for the gas volume flow in the gas line (30, 66, 96).

9. Use according to claim 8, **characterised**

  - **in that** the measuring device (22, 62, 108, 110, 124) has a data interface (42) for connecting the measuring device (22, 62, 108, 110, 124) to a volume flow sensor (44) of a house service connection, and
  - **in that** the control device (32, 64) is configured to receive the measured value for the gas volume flow in the gas line (30, 66, 96) via the data interface (42).

10. Gas distribution network (92, 122),

  - with a gas pipe system (94) of interconnected gas pipes (30, 66, 96) and
  - with a measuring device (22, 62, 108, 110, 124) for determining the calorific value of a gas flow (31, 67) in a gas line (30, 66, 96),
  - wherein the measuring device has a calorific value sensor (34, 68) which is configured to measure a value for the gas calorific value of a gas in a gas line (30, 66, 96),

  - wherein the measuring device has a control device (32, 64) which is configured to determine a value for the calorific value of the gas flow (31, 67) from the measured value for the gas calorific value and optionally further obtained measured values relating to the gas flow (31, 67), and
  - wherein the measuring device (22, 62, 108, 110, 124) is arranged and configured to determine the calorific value of a gas flow (31, 67) through one of the gas lines (30, 66, 96) of the gas line system (94),

  **characterised in that**

  - **in that** a decentralised gas feed-in system (100) is connected to a gas line (30, 66, 96) of the gas line system (94), wherein the measuring device (22, 62, 110, 124) is configured to determine the calorific value of the gas flow (31, 67) fed into the gas line system (94) by the decentralised gas feed-in system (100),
  - **in that** the control device is configured to determine information about the composition of the gas flow, namely about the hydrogen content, from the determined value for the calorific value, and
  - **in that** the decentralised gas feed-in system (100) is configured to control the gas feed into the gas line system (94) as a function of the calorific value of the gas flow determined by the measuring device (22, 62, 110, 124), namely to reduce or stop the gas feed into the gas line system if the hydrogen content determined from the determined value for the calorific value exceeds a predetermined maximum limit.

11. Gas distribution network according to claim 10, **characterised in that** a gas recipient (98) is connected to a gas line (30, 66, 96) of the gas line system (94), in particular via a house service connection, wherein the measuring device (22, 62, 110, 124) is configured to determine the calorific value of the gas flow (31, 67) supplied to the gas recipient (98).

12. Gas distribution network according to claim 11, **characterised in that** the gas recipient (98) is configured to control the gas take from the gas line system (94) or a combustion process operated with the taken gas depending on the calorific value of the gas flow determined by the measuring device (22, 62, 110, 124).

**Revendications**

1. Utilisation d'un dispositif de mesure (22, 62, 108, 110, 124) pour déterminer le pouvoir calorifique d'un flux de gaz (31, 67) dans une conduite de gaz (30, 66, 96) d'un réseau de distribution de gaz (92, 122),

- dans laquelle le dispositif de mesure comprend un capteur de pouvoir calorifique (34, 68) qui est configuré pour mesurer une valeur pour le pouvoir calorifique de gaz d'un gaz dans une conduite de gaz (30, 66, 96), et

- dans laquelle le dispositif de mesure présente un dispositif de commande (32, 64) qui est configuré pour déterminer une valeur pour le pouvoir calorifique du flux de gaz (31, 67) à partir de la valeur mesurée pour le pouvoir calorifique de gaz et, en option, d'autres valeurs de mesure obtenues pour le flux de gaz (31, 67),

- dans laquelle le réseau de distribution de gaz (92, 122) comprend un système de conduites de gaz (94) de conduites de gaz (30, 66, 96) reliées entre elles, et

- dans laquelle le dispositif de mesure (22, 62, 108, 110, 124) est disposé et configuré pour déterminer le pouvoir calorifique d'un flux de gaz (31, 67) à travers l'une des conduites de gaz (30, 66, 96) du système de conduites de gaz (94),

**caractérisée**

- **en ce qu'**une installation d'injection de gaz décentralisée (100) est raccordée à une conduite de gaz (30, 66, 96) du système de conduites de gaz (94), le dispositif de mesure (22, 62, 110, 124) étant configuré pour déterminer le pouvoir calorifique du flux de gaz (31, 67) injecté par l'installation d'injection en gaz décentralisée (100) dans le système de conduites de gaz (94)

- **en ce que** le dispositif de commande est configuré pour déterminer, à partir de la valeur déterminée pour le pouvoir calorifique, une information sur la composition du flux de gaz, à savoir sur la teneur en hydrogène, et

- **en ce que** l'installation d'injection de gaz décentralisée (100) est configuré pour commander l'injection de gaz dans le système de conduites de gaz (94) en fonction du pouvoir calorifique du flux de gaz déterminé par le dispositif de mesure (22, 62, 110, 124), à savoir pour réduire ou arrêter l'injection de gaz dans le système de conduites de gaz lorsque la teneur en hydrogène déterminée à partir de la valeur déterminée pour le pouvoir calorifique dépasse une limite maximale prédéterminée.

2. Utilisation selon la revendication 1, **caractérisée**

- **en ce que** on utilise comme capteur de pouvoir calorifique (34, 68) une pellistor ou un autre capteur de gaz pour la détection de substances gazeuses, en particulier un capteur de gaz qui transforme l'information thermique et/ou chimique du gaz à mesurer en un signal utilisable électriquement.

3. Utilisation selon la revendication 1 ou 2, **caractérisée**

- **en ce que** le dispositif de mesure (22, 62, 110, 124) est configuré pour obtenir une valeur mesurée pour la pression de gaz dans la conduite de gaz (30, 66, 96), et

- **en ce que** le dispositif de commande (32, 64) est en outre configuré pour déterminer la valeur pour le pouvoir calorifique du flux de gaz (31, 67) en fonction de la valeur mesurée pour la pression du gaz.

4. Utilisation selon la revendication 3, **caractérisée**

- **en ce que** le dispositif de mesure (22, 62, 108, 110, 124) comprend un capteur de pression qui est configuré pour mesurer une valeur pour la pression de gaz dans la conduite de gaz (30, 66, 96).

5. Utilisation selon l'une des revendications 1 à 4, **caractérisé**

- **en ce que** le dispositif de mesure (22, 62, 108, 110, 124) est configuré pour obtenir une valeur mesurée pour la température du gaz dans la conduite de gaz (30, 66, 96), et

- **en ce que** le dispositif de commande (32, 64) est en outre configuré pour déterminer la valeur pour le pouvoir calorifique du flux de gaz (31, 67) en fonction de la valeur mesurée pour la température du gaz.

6. Utilisation selon la revendication 5, **caractérisé**

- **en ce que** le dispositif de mesure (22, 62, 108, 110, 124) comprend un capteur de température (38, 72) qui est configuré pour mesurer une valeur pour la température du gaz dans la conduite de gaz (30, 66, 96).

7. Utilisation selon l'une des revendications 1 à 6, **caractérisée**

- **en ce que** le dispositif de mesure (22, 62, 108, 110, 124) est configuré pour obtenir une valeur mesurée pour le débit volumétrique de gaz dans la conduite de gaz (30, 66, 96), et

- **en ce que** le dispositif de commande (32, 64) est en outre configuré pour déterminer la valeur pour le pouvoir calorifique du flux de gaz (31, 67) en fonction de la valeur mesurée pour le débit volumétrique de gaz.

**8.** Utilisation selon la revendication 7, **caractérisée**

- **en ce que** le dispositif de mesure (22, 62, 108, 110, 124) comporte un capteur de débit volumétrique (40, 74) qui est configuré pour mesurer une valeur pour le débit volumétrique de gaz dans la conduite de gaz (30, 66, 96).

**9.** Utilisation selon la revendication 8, **caractérisée**

- **en ce que** le dispositif de mesure (22, 62, 108, 110, 124) présente une interface de données (42) pour le raccordement du dispositif de mesure (22, 62, 108, 110, 124) à un capteur de débit volumique (44) d'un branchement de maison et
- **en ce que** le dispositif de commande (32, 64) est configuré pour obtenir la valeur mesurée pour le débit volumétrique de gaz dans la conduite de gaz (30, 66, 96) via l'interface de données (42).

**10.** Réseau de distribution de gaz (92, 122),

- avec un système de conduites de gaz (94) des conduites de gaz (30, 66, 96) reliées entre elles et
- avec un dispositif de mesure (22, 62, 108, 110, 124) pour déterminer le pouvoir calorifique d'un flux de gaz (31, 67) dans une conduite de gaz (30, 66, 96),
- dans lequel le dispositif de mesure comprend un capteur de pouvoir calorifique (34, 68) configuré pour mesurer une valeur pour le pouvoir calorifique de gaz d'un gaz dans une conduite de gaz (30, 66, 96),
- dans lequel le dispositif de mesure comprend un dispositif de commande (32, 64) qui est configuré pour déterminer, à partir de la valeur mesurée pour le pouvoir calorifique de gaz et, en option, d'autres valeurs de mesure obtenues pour le flux de gaz (31, 67), une valeur pour le pouvoir calorifique du flux de gaz (31, 67), et
- dans lequel le dispositif de mesure (22, 62, 108, 110, 124) est disposé et configuré pour déterminer le pouvoir calorifique d'un flux de gaz (31, 67) à travers l'une des conduites de gaz (30, 66, 96) du système de conduites de gaz (94), **caractérisée**
- **en ce qu'**une installation d'injection de gaz décentralisée (100) est raccordée à une conduite de gaz (30, 66, 96) du système de conduites de gaz (94), le dispositif de mesure (22, 62, 110, 124) étant configuré pour déterminer le pouvoir calorifique du flux de gaz (31, 67) injecté dans le système de conduites de gaz (94) par l'installation d'injection de gaz décentralisée (100),

- **en ce que** le dispositif de commande est configuré pour déterminer, à partir de la valeur déterminée pour le pouvoir calorifique, une information sur la composition du flux de gaz, à savoir sur la teneur en hydrogène, et
- **en ce que** l'installation d'injection de gaz décentralisée (100) est configuré pour commander l'injection de gaz dans le système de conduites de gaz (94) en fonction du pouvoir calorifique du flux de gaz déterminé par le dispositif de mesure (22, 62, 110, 124), à savoir pour réduire ou arrêter l'injection de gaz dans le système de conduites de gaz lorsque la teneur en hydrogène déterminée à partir de la valeur déterminée pour le pouvoir calorifique dépasse une limite maximale prédéterminée.

**11.** Réseau de distribution de gaz selon la revendication 10, **caractérisé en ce qu'**un consommateur de gaz (98) est raccordé à une conduite de gaz (30, 66, 96) du système de conduites de gaz (94), notamment par l'intermédiaire d'un branchement de maison, le dispositif de mesure (22, 62, 110, 124) étant configuré pour déterminer le pouvoir calorifique du flux de gaz (31, 67) acheminé vers le consommateur de gaz (98).

**12.** Réseau de distribution de gaz selon la revendication 11, **caractérisé en ce que** le consommateur de gaz (98) est configuré pour commander le prélèvement de gaz dans le système de conduites de gaz (94) ou un processus de combustion fonctionnant avec le gaz prélevé en fonction du pouvoir calorifique du flux de gaz déterminé par le dispositif de mesure (22, 62, 110, 124).

Fig. 1
(Stand der Technik)

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19921167 A1
- DE 102016014151 A1
- DE 69316643 T2
- EP 2045507 A1